# EUROPÄISCHE PATENTSCHRIFT

(11) **EP 3 552 667 B1**
(45) Veröffentlichungstag und Bekanntmachung des Hinweises auf die Patenterteilung: **18.11.2020**
(21) Anmeldenummer: 18166268.5
(22) Anmeldetag: 09.04.2018
(51) Int. Cl.: A61K 8/9789, A61Q 5/00

(54) **ZUSAMMENSETZUNG ZUM ENTFERNEN VON ÜBERSCHÜSSIGEM HAARFÄRBEMITTEL**
COMPOSITION FOR REMOVING EXCESS HAIR DYE
COMPOSITION POUR ENLEVER L'EXCÈS DE TEINTURE CAPILLAIRE

(43) Veröffentlichungstag der Anmeldung: 16.10.2019
(73) Patentinhaber: Demirkol, Yonca, 41238 Mönchengladbach (DE)
(72) Erfinder: DEMIRKOL, Cevat, 41238 Mönchengladbach (DE)
(74) Vertreter: Fabry, Bernd

(56) Entgegenhaltungen:
- US-A1- 2006 293 207
- DATABASE WPI Thomson Scientific, London, GB; AN 2013-S81501 XP002782206, "Hair dye stain remover for skin, clothes, fiber material, and cotton material comprises Cassia nomame powder, coconut oil fatty acid, cigarette ash, vitamin C, glycerol, and essence", & CN 103 191 043 A (ZHANG Y) 10. Juli 2013 (2013-07-10)

## Beschreibung

### GEBIET DER ERFINDUNG

Die Erfindung befindet sich auf dem Gebiet der Kosmetik, speziell der Haarkosmetik und betrifft unter anderem eine Zubereitung mit natürlichen Wirkstoffen, mit deren Hilfe man ohne die Gefahr von Hautirritationen Farbe, die beim Färben oder Tönen der Haare an die Kopfhaut gelangt ist, entfernen kann.

### TECHNOLOGISCHER HINTERGRUND

Schon vor 3.500 Jahren galt es als schick, sich das Haupthaar zu kolorieren - und zwar nicht nur unter Frauen, denn auch Männer färbten sich gerne mal den Bart. Damals war zum Beispiel Henna ein beliebtes Färbemittel. Im alten Rom benutzte man hingegen eine Paste aus Bleioxid und Löschkalk - frühzeitliche Nanotechnologie, wie vor einigen Jahren festgestellt wurde -, wirkungsvoll, aber vermutlich auch ziemlich gesundheitsschädlich

Zur Zeit der Germanischen Kriege kamen neue Farbwünsche dazu: Jetzt blondierten sich die Römerinnen ihr schwarzes Haar mit Buchenasche, Seife und Quittensaft - was aber oft dazu führte, dass ihnen die Haare anschließend komplett ausfielen.

Um die Jahrhundertwende gab es die ersten synthetischen Haarfarben, die als Bleichmittel Wasserstoffperoxid beinhalteten. Im Jahr 1934 meldeten die Ströher-Werke im sächsischen Oberwiesenthal eine Haarfärbe-Rezeptur zum Patent an, die in den Kriegswirren aber zunächst verloren ging. Auf Basis dieser Formulierung präsentierte das Darmstädter Unternehmen Wella dann aber 1950 die erste Creme-Haarfarbe der Welt. Inzwischen teilt sich der Markt vor allem zwischen den großen Kosmetikherstellern wie L'Oréal, Garnier, Procter&Gamble (Wella), Kao (Goldwell) und Henkel (Schwarzkopf) auf.

Heute gehören Haarfärbemittel zu den beliebtesten kosmetischen Produkten und werden von mehr als 60 % der rauen und zwischen 5 bis 10 % der Männer in Europa verwendet. In der EU müsse Haarfärbemittel den Anforderungen der EG-KosmetikVO entsprechen. Das gilt sowohl für Haarfarben, die von Friseuren verwendet werden, als auch für die Haarfärbemittel des privaten Gebrauchs.

Ein bekanntes Problem besteht darin, dass sich beim Auftragen der Haarfarben praktisch nicht vermeiden lässt, dass auch ein Teil der Farbe mit der Kopfhaut in Kontakt gelangt, was insbesondere am Haaransatz eine unerwünschte Färbung hervortreten lässt, die umso auffälliger ist, je dunkler die Farbe und je heller die Haut ist.

Üblicherweise werden diese Farbreste nach Abschluss der Haartönung oder -färbung entfernt, indem die Hautpartien mit einem organischen Reiniger behandelt werden, in dem die Haarfarbe löslich ist. Auch Zitronensaft kann zum Einsatz kommen. Diese Mittel sind jedoch vergleichsweise aggressiv und können bei empfindlichen Kunden zu einer starken Hautirritation führen.

### STAND DER TECHNIK

Gegenstand der US 2006 293207 A1 (PORTER) sind Entfernungsmittel für Haarfarben, deren Hauptbestandteil Tabakasche ist. Die Schrift offenbart weder die spezielle Mischung aus Asche von verschiedenen Tabakblättern noch die Kombination mit der Asche von Filterhülsen.

Die CN 103191043 A (ZHANG) hat ebenfalls ein Entfernungsmittel für Haarfarben von der (Kopf-)Haut zum Gegenstand, allerdings wird hier neben einer Reihe anderer Bestandteile nicht Tabakasche, sondern Zigarettenasche eingesetzt.

### AUFGABE DER ERFINDUNG

Eine erste Aufgabe der vorliegenden Erfindung hat daher darin bestanden, eine Zubereitung zur Verfügung zu stellen, die ausschließlich natürliche Wirkstoffe enthält und mit deren Hilfe man auch hartnäckige, insbesondere sehr dunkle Farbreste, die beim Tönen oder Färben von Haaren auf der Kopfhaut zurückbleiben, schnell, umfassend und irritationsfrei entfernen kann.

Eine zweite Aufgabe ist darin zu sehen, eine Darreichungsform zur Verfügung zu stellen, die insbesondere für Nutzungen außerhalb des Friseursalons dem Anwender die Möglichkeit gibt, Rückstände des Färbens von der Kopfhaut zu entfernen, ohne deshalb neben dem Färbemittel auch noch getrennt von diesem ein Entfärbemittel kaufen zu müssen.

### BESCHREIBUNG DER ERFINDUNG

Ein erster Gegenstand der Erfindung betrifft eine kosmetische Zubereitung enthaltend
(a) Asche von mindestens zwei, vorzugsweise von drei verschiedenen Tabakblättern, nämlich hellen, mittelfarbigen und dunklen, oder alternativ
(b) Tabakasche sowie Asche von Filterhülsen.

Überraschenderweise wurde gefunden, dass Asche von mindestens zwei, vorzugsweise drei verschiedenen Tabakblättern und/oder Mischungen mit der Asche von Filterhülsen, insbesondere wenn in einem neutralen flüssigen Träger suspendiert, einen idealen Wirkstoff darstellt, um Reste von synthetischen Farben, die beim Tönen oder Färben auf die Kopfhaut gelangt sind, rückstandslos, schnell und vor allem ohne jede Hautirritation zu entfernen. Dieser Effekt ist nicht an die Haarfarbe bzw. den darin enthaltenen chemischen Inhaltsstoff gebunden, insbesondere werden auch Reste von dunklen und sogar schwarzen Farben entfernt.

### TABAKASCHE

Auch wenn im Sinne der vorliegenden Erfindung jede Art von Tabakasche geeignet ist, um die Farbreste von der Kopfhaut zu entfernen, hat die Anmelderin doch überraschend gefunden, dass es zwei Ausführungsformen gibt, die anderen überlegen sind. Dazu gehört, dass die Asche von mindestens zwei, vorzugsweise von drei verschiedenen Tabakblättern, nämlich hellen, mittelfarbigen und dunklen stammt. Darunter ist nicht zwangsläufig zu verstehen, dass es sich um zwei oder drei verschiedene Tabaksorten handeln muss, die Blätter können auch von der gleichen Pflanze stammen, die Färbung macht lediglich deutlich, dass die Zusammensetzung der Inhaltsstoffe unterschiedlich ist und dementsprechend auch die Zusammensetzung, die sich nach der Veraschung im Endprodukt wiederfindet. Als besonders vorteilhaft hat es sich erwiesen, die drei Typen - hell, mittel, dunkel - im Gewichtsverhältnis (0,1-0,4):(0,1-0,4):1 einzusetzen. Besonders bevorzugt ist eine Mischung im Gewichtsverhältnis 1:1:2.

Die Anmelderin hat in diesem Zusammenhang auch Versuche mit Zigarettenasche durchgeführt. Dabei wurde gefunden, dass diese nicht nur eine schlechtere Entfärbewirkung besitzt, sondern - vermutlich aufgrund der vielen Zusatzstoffe - auch wieder hautirritierend wirkt. Der Einsatz von Zigarettenasche wird - wenngleich wesentlich billiger - von vielen Verbrauchern zudem aus hygienischen Gründen nicht akzeptiert. Gleichwohl ist es möglich, der Tabakasche bis zu 25 Gew.-% Zigarettenasche zuzusetzen, ohne einen nennenswerten Leistungsverlust zu beobachten; lediglich die Entfernung von sehr dunkler Farbe wird deutlich schwieriger.

### FILTERHÜLSEN

Der vorliegenden Erfindung liegt ferner die zufällige und daher tatsächlich völlig überraschende Erkenntnis zu Grunde, dass sich die Farbentfernung noch einmal stark verbessern lässt, wenn man Tabakasche mit der Asche von Filterhülsen mischt. Filterhülsen stellen Papierprodukte dar, die als Zigarettenzubehör im Handel in unterschiedlichsten Formen erhältlich sind (z.B. die Marken Angel, Columbus oder Energx). Es hat sich gezeigt, dass der Zusatz von 1 bis etwa 30 und insbesondere etwa 10 bis etwa 25 und insbesondere etwa 15 bis etwa 20 Gew.-% der Filterhülsenasche zur Tabakasche die besten Ergebnisse zeigt.

### ENTZÜNDUNGSHEMMENDE STOFFE

Die Zubereitungen können weiterhin entzündungshemmende Stoffe enthalten. Typische Beispiele sind Bisabolol, Allantoin, Harnstoff, Azulen, Kamillenwirkstoffe und Acetylsalicylsäurederivate.

Zu dieser Gruppe können auch physiologische Kühlstoffe gezählt werden. Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

### DUFTSTOFFE UND AROMEN

Die Zubereitungen können auch Duftstoffe und/oder Aromen enthalten, deren Auswahl jedoch so zu treffen ist, dass von ihnen keine Irritationsgefahr ausgeht.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage. Besonders bevorzugt ist Kamillenöl bzw. Kamillenextrakt, weil dieser sowohl als Aroma wirkt, als auch Hautirritationen entgegenarbeitet.

### TRÄGER

Die bevorzugte Darreichungsform der kosmetischen Zubereitungen ist die Aufschlämmung bzw. Dispersion der Tabakasche sowie gegebenenfalls der weiteren Inhaltsstoffe in einem geeigneten flüssigen Träger. Hierfür kommen insbesondere Mono-,Di- und/oder Triglyceriden, Ethylenglycol, Propylenglycol, Diethylenglycol, Dipropylenglycol und Triacetin in Betracht. Bei weitem bevorzugt ist indes Glycerin. Besteht die Formulierung nur aus Tabakasche und Träger, speziell Glycerin, so kann der Feststoffanteil zwischen 30 und 50 Gew.-% und insbesondere zwischen 35 und 40 Gew.-% liegen.

### ZUSAMMENSETZUNGEN

Im Sinne der vorliegenden Erfindung sind solche Zubereitungen bevorzugt, enthaltend oder bestehend aus:
(a) etwa 50 bis etwa 70 Gew.-% und insbesondere etwa 55 bis etwa 60 Gew.-% Träger
(b) etwa 30 bis etwa 50 Gew.-% und insbesondere etwa 40 bis etwa 45 Gew.-% Tabakasche wie oben definiert;
(c) 0 bis etwa 5 Gew.-% und insbesondere etwa 0,5 bis etwa 3 Gew.-% entzündungshemmende Stoffe; sowie
(d) 0 bis etwa 1 Gew.-% und insbesondere etwa 0,2 bis etwa 0,5 Gew.-% Duftstoffe und/oder Aromen,
   mit der Maßgabe, dass sich die Mengenangaben gegebenenfalls mit weiteren Hilfs- und Zusatzstoffen zu 100 Gew.-% ergänzen.

Alternativ bevorzugt sind Zubereitungen enthaltend oder bestehend aus
(a) etwa 50 bis etwa 70 Gew.-% und insbesondere etwa 55 bis etwa 60 Gew.-% Träger
(b) etwa 30 bis etwa 50 Gew.-% und insbesondere etwa 40 bis etwa 45 Gew.-% einer Mischung von Tabakasche wie oben definiert und Asche von Filterhülsen;
(c) 0 bis etwa 5 Gew.-% und insbesondere etwa 0,5 bis etwa 3 Gew.-% entzündungshemmende Stoffe; sowie
(d) 0 bis etwa 1 Gew.-% und insbesondere etwa 0,2 bis etwa 0,5 Gew.-% Duftstoffe und/oder Aromen,
   mit der Maßgabe, dass sich die Mengenangaben gegebenenfalls mit weiteren Hilfs- und Zusatzstoffen zu 100 Gew.-% ergänzen.

Die Komponente (b) enthält dabei die Tabakasche und die Asche der Filterhülsen vorzugsweise im Gewichtsverhältnis von etwa 99:1 bis etwa 70:30 und insbesondere von etwa 90:10 bis etwa 80:20.

### GEWERBLICHE ANWENDBARKEIT

Ein weiterer Gegenstand der Erfindung betrifft ein kosmetisches Verfahren zur Entfernung von überschüssiger Farbe nach der Haarfärbung von der Kopfhaut, umfassend oder bestehend aus den folgenden Schritten:
(i) Bereitstellen einer kosmetischen Zubereitung wie vorstehend beschrieben; sowie
(ii) Inkontaktbringen und Behandeln der verfärbten Hautfläche mit der Zubereitung aus Schritt (I).

Ein weiterer Gegenstand der Erfindung betrifft die Verwendung einer kosmetischen Zubereitung wie vorstehend beschrieben zur Entfernung von überschüssiger Farbe bei der Haarfärbung von der Kopfhaut. Dabei besteht die bevorzugte Ausführungsform darin, ein wenig des Produktes, also der Tabakaschensuspension, auf einen Wattebausch zu geben und die verfärbten Hautpartien abzutupfen.

Ein letzter Gegenstand der Erfindung umfasst ein Kit bestehend aus einer ersten Container, die die Haarfarbe enthält und einem zweiten Behältnis mit der vorstehend beschriebenen Farbentfernungszubereitung. Die beiden Aufbewahrungsformen können gemeinsam in einer Verpackung angeboten werden oder aber als Doppelpack. Die Haarfarbe kann dabei sowohl als Paste in einer Tube vorliegen oder auch als flüssiges Produkt.

### BEISPIELE

### BEISPIEL 1 BIS 10, VERGELICHSVERSUCHE V1 BIS V4

Es wurden Zubereitungen hergestellt, bei denen jeweils 40 g Feststoff zu 60 g Glycerin gegeben und homogenisiert wurden. Für den Vergleichsversuch V2 wurde reiner Zitronensaft verwendet. Jeweils etwa 10 g der so erhaltenen Tinkturen (bei V2 etwa 5 g) wurden auf jeweils einen Wattebausch gegeben und die Farbreste nach Färbung mit verschiedenen Permamenthaarfärbeprodukten abgetupft. Folgende Haarfarben wurden eingesetzt:
A Excellence Creme (schwarz)
B Excellence Creme (kühles kastanienbraun)
C Casting Creme Gloss (schwarzbraun)
D Casting Crème Gloss (dunkle Kirsche)

Alle Produkte sind von der Firma L'Oréal im Handel erhältlich. Die Ergebnisse sind in den **Tabellen 1 und 2** wiedergegeben. Beurteilt wurden das rückstandsfreie Entfernen (1 = zweifaches Tupfen genügt; 2 = mehrfaches Tupfen erforderlich; 3 = Reiben erforderlich; 4 = Farbreste trotz Reiben) und die Hautirritation (0 = keine; 1 = leichte Rötung; 2 = leichtes Brennen).

**Tabelle 1**

| Entfernung von Haarfarbresten | | | | | |
|---|---|---|---|---|---|
| **Bsp.** | **Wirkstoff** | **A** | **B** | **C** | **D** |
| 1 | 100 % Tabakasche A¹ | 2 | 2 | 2 | 2 |
| 2 | 100 % Tabakasche B² | 2 | 1 | 2 | 1 |
| 3 | 80 % Tabakasche A + 20 % Filterasche | 2 | 1 | 2 | 1 |
| 4 | 80 % Tabakasche B + 20 % Filterasche | 1 | 1 | 1 | 1 |
| 5 | 90 % Tabakasche B + 10 % Zigarettenasche | 2 | 2 | 2 | 2 |
| V1 | 100 % Zigarettenasche | 4 | 3 | 4 | 3 |
| V2 | 100 % Zitronensaft | 4 | 3 | 4 | 3 |

| | | | | | |
|---|---|---|---|---|---|
| 1) Asche ausschließlich auf Grundlage von sehr dunklen Tabakblättern 2) Asche auf Grundlage von hellen, mittel gefärbten und dunklen Tabakblättern (Gewichtsverhältnis 1:1:2) | | | | | |

**Tabelle 2**

| Hautirritation | | | | | |
|---|---|---|---|---|---|
| **Bsp.** | **Wirkstoff** | **A** | **B** | **C** | **D** |
| 6 | 100 % Tabakasche A¹ | 0 | 0 | 0 | 0 |
| 7 | 100 % Tabakasche B² | 0 | 0 | 0 | 0 |
| 8 | 80 % Tabakasche A + 20 % Filterasche | 0 | 0 | 0 | 0 |
| 9 | 80 % Tabakasche B + 20 % Filterasche | 0 | 0 | 0 | 0 |
| 10 | 90 % Tabakasche B + 10 % Zigarettenasche | 0 | 0 | 0 | 0 |
| V3 | 100 % Zigarettenasche | 1 | 1 | 1 | 1 |
| V4 | 100 % Zitronensaft | 2 | 2 | 2 | 2 |

Die Beispiele und Vergleichsbeispiele zeigen, dass mit den erfindungsgemäßen Tabakasche-Suspensionen Farbreste auch gerade von besonders dunklen Farben rückstandsfrei entfernt werden können, ohne dass es zu Irritationen der empfindlichen Kopfhaut kommt. Dabei schneidet das Produkt auf Basis der Tabakmischung in Kombination mit der Asche von Filterhülsen am besten ab. Reine Zigarettenasche erweist sich als deutlich schlechter, insbesondere bei schwarze Farbe bleiben trotz Reiben häufig Farbreste zurück; zudem kommt es zu leichter Hautrötung. Noch weniger empfehlenswert ist das "Hausmittel" Zitronensaft: schwarze Farben werden nicht rückstandsfrei entfernt und es kommt nicht nur zu Hautrötungen, sondern auch zum Brennen der Kopfhaut.

### FORMULIERUNGSBEISPIELE F1 BIS F6

| **Wirkstoff** | **F1** | **F2** | **F3** | **F4** | **F5** | **F6** |
|---|---|---|---|---|---|---|
| 80 % Tabakasche A + 20 % Filterasche | 30 | - | 24.4 | 24.4 | 29.9 | - |
| 80 % Tabakasche B + 20 % Filterasche | - | 30 | - | - | - | 29.9 |
| Bisabolol | - | - | 0,5 | - | - | - |
| Allantoin | - | - | - | 0,5 | - | - |
| Kamillenextrakt | - | - | 0,1 | 0,1 | 0,1 | 0,1 |
| Glycerin | 70 | 70 | 70 | 70 | | |
| Ethylenglykol | - | - | - | | 70 | 70 |

## Patentansprüche

1. Kosmetische Zubereitung, enthaltend Asche von mindestens zwei, vorzugsweise von drei verschiedenen Tabakblättern, nämlich hellen, mittelfarbigen und dunklen.

2. Kosmetische Zubereitung, enthaltend Tabakasche sowie Asche von Filterhülsen.

3. Zubereitung nach den Ansprüchen 1 und/oder 2, **dadurch gekennzeichnet, dass** die Tabakasche bis zu 25 Gew.-% Zigarettenasche enthält.

4. Zubereitung nach den Ansprüchen 1 und/oder 2, **dadurch gekennzeichnet, dass** die Tabakasche 1 bis 30 Gew.-% Asche von Filterhülsen enthält.

5. Zubereitung nach mindestens einem der Ansprüche 1 bis 4, **dadurch gekennzeichnet, dass** sie weiterhin entzündungshemmende Stoffe enthält.

6. Zubereitung nach mindestens einem der Ansprüche 1 bis 5, **dadurch gekennzeichnet, dass** sie weiterhin Duftstoffe und/oder Aromen enthält.

7. Zubereitung nach mindestens einem der Ansprüche 1 bis 6, **dadurch gekennzeichnet, dass** sie weiterhin einen flüssigen Träger enthält.

8. Zubereitung nach Anspruch 7, **dadurch gekennzeichnet, dass** der flüssige Träger ausgewählt ist aus der Gruppe, die gebildet wird von Glycerin, Mono-,Di- und/oder Triglyceriden, Ethylenglycol, Propylenglycol, Diethylenglycol, Dipropylenglycol und Triacetin.

9. Zubereitung nach mindestens einem der Ansprüche 1 bis 8, enthaltend oder bestehend aus:
(a) 50 bis 70 Gew.-% Träger
(b) 30 bis 50 Gew.-% Tabakasche;
(c) 0 bis 5 Gew.-% entzündungshemmende Stoffe; sowie
(d) 0 bis 1 Gew.-% Duftstoffe und/oder Aromen, mit der Maßgabe, dass sich die Mengenangaben gegebenenfalls mit weiteren Hilfs- und Zusatzstoffen zu 100 Gew.-% ergänzen.

10. Zubereitung nach mindestens einem der Ansprüche 1 bis 8, enthaltend oder bestehend aus:
(a) 50 bis 70 Gew.-% Träger
(b) 30 bis 50 Gew.-% einer Mischung von Tabakasche und Asche von Filterhülsen;
(c) 0 bis 5 Gew.-% entzündungshemmende Stoffe; sowie
(d) 0 bis 1 Gew.-% Duftstoffe und/oder Aromen; mit der Maßgabe, dass sich die Mengenangaben gegebenenfalls mit weiteren Hilfs- und Zusatzstoffen zu 100 Gew.-% ergänzen.

11. Zubereitung nach Anspruch 10, **dadurch gekennzeichnet, dass** die Komponente (b) die Tabakasche und die Asche der Filterhülsen im Gewichtsverhältnis von 99:1 bis 70:30 enthält.

12. Kosmetisches Verfahren zur Entfernung von überschüssiger Farbe nach der Haarfärbung von der Kopfhaut, umfassend oder bestehend aus den folgenden Schritten:
(i) Bereitstellen einer kosmetischen Zubereitung nach einem der Ansprüche 1, 2, 9 oder 10,
(ii) Inkontaktbringen und Behandeln der verfärbten Hautfläche mit der Zubereitung aus Schritt (i).

13. Verwendung einer kosmetischen Zubereitung nach mindestens einem der Ansprüche 1 bis 11 zur Entfernung von überschüssiger Farbe bei der Haarfärbung von der Kopfhaut.

14. Kit bestehend aus einem ersten Container mit der Haarfarbe und einem zweiten Behältnis enthaltend die kosmetische Zubereitung nach mindestens einem der Ansprüche 1 bis 11.

## Claims

1. Cosmetic preparation containing ashes of at least two, preferably three, different tobacco leaves, namely light, medium and dark colored.

2. Cosmetic preparation containing tobacco ash and ash from filter tubes.

3. Preparation according to claims 1 and/or 2, **characterized in that** the tobacco ash contains up to 25% by weight of cigarette ash.

4. Preparation according to claims 1 and/or 2, **characterized in that** the tobacco ash contains 1 to 30% by weight of ash from filter tubes.

5. Preparation according to at least one of claims 1 to 4, **characterized in that** it further contains anti-inflammatory substances.

6. Preparation according to at least one of claims 1 to 5, **characterized in that** it further contains fragrances and/or flavors.

7. Preparation according to at least one of claims 1 to 6, **characterized in that** it further contains a liquid carrier.

8. Preparation according to claim 7, **characterized in that** the liquid carrier is selected from the group consisting of glycerol, mono-, di- and/or triglycerides, ethylene glycol, propylene glycol, diethylene glycol, dipropylene glycol and triacetin.

9. Preparation according to at least one of claims 1 to 8, containing or consisting of:
(a) from about 50 to about 70% by weight of carrier;
(b) about 30 to about 50 % by weight tobacco ash;
(c) 0 to about 5% by weight of anti-inflammatory substances; and
(d) 0 to about 1% by weight of fragrances and/or flavors,
with the proviso that the quantities indicated may add to 100 % by weight with other auxiliaries and additives.

10. Preparation according to at least one of claims 1 to 8, containing or consisting of:
(a) about 50 to about 70 % by weight of carrier;
(b) from about 30% to about 50% by weight of a mixture of tobacco ash and filter tube ash;
(c) 0 to about 5% by weight of anti-inflammatory substances; and
(d) 0 to about 1% by weight of fragrances and/or flavors;
with the proviso that the quantities indicated add to 100% by weight with further auxiliaries and additives.

11. Preparation according to claim 10, **characterized in that** component (b) contains the tobacco ash and the ash of the filter tubes in a weight ratio of about 99:1 to about 70:30.

12. Cosmetic process for removing excess color after hair coloration from the scalp, comprising or consisting of the following steps:
(i) providing a cosmetic preparation according to any one of Claims 1, 2, 9 or 10,
(ii) Contacting and treating the discolored skin area with the preparation from step (i).

13. Use of a cosmetic preparation according to at least one of claims 1 to 11 for removing excess color in hair coloration from the scalp.

14. Kit consisting of a first container containing the hair color and a second container containing the cosmetic preparation according to at least one of claims 1 to 11.

## Revendications

1. Préparation cosmétique contenant des cendres d'au moins deux, de préférence trois feuilles de tabac différentes, à savoir de couleur claire, moyenne et foncée

2. Préparation cosmétique contenant de la cendre de tabac et de la cendre provenant de tubes filtres

3. Préparation selon les revendications 1 et/ou 2, **caractérisée en ce que** la cendre de tabac contient jusqu'à 25 % en poids de cendre de cigarette.

4. Préparation selon les revendications 1 et/ou 2, **caractérisée en ce que** la cendre de tabac contient 1 à 30 % en poids de cendres provenant de tubes filtrants.

5. Préparation selon au moins une des revendications 1 à 4, **caractérisée en ce qu'**elle contient en outre des substances anti-inflammatoires.

6. Préparation selon au moins une des revendications 1 à 5, **caractérisée en ce qu'**elle contient en outre des parfums et/ou des arômes.

7. Préparation selon au moins une des revendications 1 à 6, **caractérisée en ce qu'**elle contient en outre un support liquide.

8. Préparation selon la revendication 7, **caractérisée en ce que** le support liquide est choisi dans le groupe constitué par le glycérol, les mono-, di- et/ou triglycérides, l'éthylène glycol, le propylène glycol, le diéthylène glycol, le dipropylène glycol et la triacétine.

9. Préparation selon au moins une des revendications 1 à 8, contenant ou consistant en :
(a) d'environ 50 à environ 70 % en poids du transporteur
(b) d'environ 30 à 50 % en poids de cendres de tabac ;
(c) 0 à environ 5 % en poids de substances anti-inflammatoires ; et
(d) 0 à environ 1 % en poids de parfums et/ou d'arômes,
sous réserve que les quantités indiquées puissent être complétées, le cas échéant, par d'autres auxiliaires et additifs à concurrence de 100 % en poids

10. préparation selon au moins une des revendications 1 à 8, contenant ou consistant en :
(a) d'environ 50 à environ 70 % en poids du transporteur
b) d'environ 30 % à environ 50 % en poids d'un mélange de cendres de tabac et de cendres de tubes filtrants ;
(c) 0 à environ 5 % en poids de substances anti-inflammatoires ; et
(d) 0 à environ 1 % en poids de parfums et/ou d'arômes ;
à condition que les quantités indiquées puissent être complétées à 100% en poids par d'autres auxiliaires et additifs.

11. Préparation selon la revendication 10, **caractérisée en ce que** le composant (b) contient la cendre de tabac et la cendre des tubes filtrants dans un rapport pondéral d'environ 99:1 à environ 70:30.

12. Procédé cosmétique pour éliminer du cuir chevelu l'excès de couleur après la teinture des cheveux, comprenant ou consistant en les étapes suivantes
(i) fournir une préparation cosmétique selon l'une des revendications 1, 2, 9 ou 10,
(ii) contacter et traiter la zone décolorée de la peau avec la préparation de l'étape (i).

13. Utilisation d'une préparation cosmétique selon au moins une des revendications 1 à 11 pour éliminer l'excès de couleur du cuir chevelu lors de la coloration des cheveux.

14. Kit constitué d'un premier récipient contenant la couleur de cheveux et d'un deuxième rapport contenant la préparation cosmétique selon au moins une des revendications 1 à 11.
